# EUROPEAN PATENT APPLICATION

(11) **EP 0 724 842 A2**
(43) Date of publication of application: **07.08.1996**
(21) Application number: 95830519.5
(22) Date of filing: 14.12.1995
(51) Int. Cl.: A23L 1/29

(54) **Preparation for the treatment of obesity**

(30) Priority: 15.12.1994 IT RM940806
(71) Applicant: Picarelli, Antonio, 00144 Roma (IT)
(72) Inventor: Picarelli, Antonio, 00144 Roma (IT)
(74) Representative: Sarpi, Maurizio

(57) **Abstract**

There is disclosed a preparation having a low calorie content and aiming at causing both the biliary and pancreatic secretions and the emptying of the cholecyst so as to reduce, before meals, the common reserves of biliary salts and enzymes present in the organism and adapted to initiate a regular digestion process. The preparation includes fats, proteins, and sugars with the addition of a biliary salt such as sodium taurocholate.

## Description

The present invention relates to a product for the treatment of obesity and dislipoprotidemia which takes its effect on the digestion and reduces the capability of the patient to digest the swallowed food.

It is known that the capability of storing chemical energy from foods allows a significant increase of the surviving capacity of the human bodies living under hard environmental conditions due to the difficulty of being provided with a certain quantity of food in the time. On the other hand, the Western society are not presently influenced by periodic, insufficient food quantity, but are instead continuously provided with foodstuffs beyond necessary. Accordingly, such a capability of storing energy under conditions of large quantity of foods is a negative factor and causes obesity. By obesity it is meant an excess of adipose tissue in the organism. The meaning of excess is rather difficult to be defined. In the clinical practice, easy anthropometrical measures put the weight of the subject to be examined in relation with a medium sample of people sorted by weight and age. As the increase of the adipose component in the organism does not necessarily involve disease conditions, it is difficult to define the limit beyond which the accumulation of fat assumes a pathological meaning. Conventionally, that limit is fixed to about 20% of the ideal weight. By ideal weight it is meant the medium weight of people comparable to the subject to be examined by sex, age, and ethnic group of belonging.

In any case the obesity is to be considered as a circumstance predisposing and aggravating the course of other pathological disorders. The study of Framingham proved that an excess of 20% over the ideal weight implies certain risks for the state of health. According to recent data, which are probably estimated by defect, 20-30% of men and 30-40% of women are obese.
The pathological conditions more frequently associated to the obesity are: arterial hypertension, atherosclerosis, cardiovascular diseases, particularly coronary insufficiency, and diabetes mellitus. All of such pathological conditions affect directly Western people and the annual cost of the therapeutics is very high.
Hitherto the treatment of the obesity is not satisfactory both for the patient and the doctor. Restriction on calories (diet) is certainly the corner-stone of any weight reduction program. The principle, on which such a practice is based, is easy: if the supply of calories is lower than the daily requirements of the organism, the energy reserve of the organism stored under the form of fat will be used for the energy balance, and then will be reduced. Corollary of such a strategy is the physical exercise as it is capable of increasing the energy waste of the organism and then increasing the use of the energy reserve of the same.
The obesity has became a multi-millionaire affair in the Western countries owing to a variety of more or less fanciful and/or scientific diets. Moreover such a situation is assisted not only by the wide spread of the relative semi-pathological condition but also and above all by the lack of a lasting result of the dietetic therapies.

For those reasons mainly due to bad alimentary habits, the phytotherapy, which has further increased the already high cost of the dietetic therapies, was proposed. The major problem of the obesity treatment is not to lose weight but to keep the achieved weight. Besides the restriction on calories also two classes of drugs are used in the treatment of the obesity: the drugs for the treatment of the anorexia and the thyroidal hormones. In addition to the lack of a practical use in the physiopathological field such two classes of drugs are contra-indicated and potentially harmful in case of chronic use. The thyroidal hormones lead to a negative nitrogen balance and are potentially cardiotoxic except for those subjects with a clear hypothyroidism. The drugs for the treatment of anorexia, such as amphetamine, which are effective only for short periods of time, exert an exciting action on the central nervous system and can cause a number of important side-effects (palpitations, arterial hypertension, a.s.o.) and tolerance problems due to their excessive use.

The present invention seeks to solve such problems by providing a preparation for the treatment of obesity which takes effect directly on the digestion, as it reduces temporarily the capability of the patient to fully digest the swallowed foods, and further gives no tolerance problems and has no contra-indications.

According to the invention this is achieved by a dietetic product which can be administered before eating and is capable to physiologically stimulate both the biliary and pancreatic secretions and the cholecyst contraction so that, upon swallowing foods, the organism does not have those reserves of biliary salts and enzymes adapted to initiate the digestion process.
Summing up, it was noticed that upon swallowing before meals a preparation having a low calorie content and aiming at causing both the biliary and pancreatic secretions and the emptying of the cholecyst, the patient will not be able to regularly digest, as usual, the foods which he will eat during a determined period of time after the administration of the product, as the organism is lacking in bile and pancreatic juice which have been voluntarily wasted during an "useless" digestion.
Since the digestion capability is exhausted, the amount of material which can be absorbed is consequently reduced, with the result of a reduction of both the calories, causing a loss of weight, and the quantity of fats and glucides absorbed, which takes effect on the lipemia (cholesterol and triglycerides).
Under such conditions, therefore, even foods having a high calorie content will supply the patient with a reduced amount of calories, with the result that the patient can keep to a normal diet without absolutely reducing the quantity of the foods and/or without having to select the quality thereof according to the energy requirements.
In order that the preparation takes its effect in short time so that the waiting time between the administration and the meals can be reduced, it is of course desired that the preparation can be administered in liquid form and in a sufficient volumetric quantity to allow the active substances to reach the duodenum quite soon. Additionally, the total calories of the effective dietetic dose will have to be small, in any case not higher than 80-100 kcal.

Through a number of tests a preparation has been found which is capable to cause the desired effects and to satisfy the requirements mentioned above without any contra-indication.
According to the invention such a preparation includes generally fats, proteins and sugars, each of such nutrients being present at least in the minimum amount able to stimulate the digestion capability.
More specifically the above-mentioned nutrients are:
fatty acids with c₁₈,
essential aminoacids,
carbohydrate in form of monosaccharides,
to which a biliary salt or other cholagogic and/or choleretic substance is added in order to increase the contraction of the cholecyst and the production of bile. A preferred formulation, which is mentioned by way of example and keeps into account the literature regarding the minimum amount of such nutrients to stimulate the secretory functions, is the following:
Oleic acid: 2% by weight
Phenylalanine-L; Tryptophan; Methionine; Valine:
0,001% by weight
D-dextrose: 6% by weight
Sodium taurocholate: 0,3% by weight.
Such formulation is carried by about 100-150 cm³ of water with a conveniently corrected pH of 2 to 3.
An easy calculation of the calorie content comes to the conclusion that such a proportioning has at most 80 kcal, i.e. a not important energy contribution.

The effects of the described product were tested in a number of patients who followed treatments of some months without any contra-indication, with important results both as far as losing weight and keeping the achieved weight is concerned.

A preferred embodiment of the preparation for the treatment of obesity has been described. A number of modifications and changes of the same will be evident to those skilled in the art. Therefore, it should be understood that the present invention can be embodied in a different manner from that specifically described within the scope of the appended claims.

## Claims

1. Dietetic preparation for the treatment of obesity, wherein it includes fats, proteins and sugars, each of such nutrients being present in the minimum amount necessary to stimulate the digestion capability of the patient before meals through the biliary and pancreatic secretions so as to reduce, before meals, the common reserves of biliary salts and enzymes present in the organism and adapted to initiate a regular digestion process.

2. Preparation for the treatment of obesity according to claim 1, wherein it is supplied in form of a liquid mixture of sufficient volume to allow the active substances to reach the duodenum as soon as possible in order to reduce the waiting time between the administration of the product and the meal.

3. Preparation for the treatment of obesity according to the preceding claims, wherein the calorie amount of the minimum effective dose is between 40 and 80 kcal.

4. Preparation for the treatment of obesity according to the preceding claims, wherein the fatty acids are C₁₈ fatty acids.

5. Preparation for the treatment of obesity according to the preceding claims, wherein the proteins are supplied under the form of essential aminoacids.

6. Preparation for the treatment of obesity according to the preceding claims, wherein the sugars are represented by D-dextrose.

7. Preparation for the treatment of obesity according to the preceding claims, wherein a substance capable to increase the cholecyst contraction and the bile production is added thereto.

8. Preparation for the treatment of obesity according to claim 7, wherein said substance is a biliary salt such as sodium taurocholate.

9. Preparation for the treatment of obesity according to the preceding claims, comprising:
oleic acid in the range of 2 to 4% by weight,
phenylalanine-L, tryptophan, methionine, and valine alone or in combination in the range of 0,001 to 0,002% by weight,
D-dextrose in the range of 6 to 8% by weight.

10. Preparation for the treatment of obesity according to claim 9, comprising water as excipient in the range of 100 to 150 cm³.

11. Preparation for the treatment of obesity according to claims 9 and 10, wherein sodium taurocholate in the range of 0,3 to 0,5% by weight is added thereto.

12. Preparation for the treatment of obesity according to claims 9 to 11, wherein the total calorie amount is in the range of 40 to 100 kcal.

13. Preparation for the treatment of obesity according to claims 9 to 12, wherein the final liquid mixture is brought to a pH of 2 to 3.

14. Use of sodium taurocholate in a dietetic preparation for the treatment of obesity, comprising fats, proteins, and sugars present in a sufficient quantity to stimulate the digestion capability in order to increase the cholecyst contraction and the bile production.
